# EUROPEAN PATENT SPECIFICATION

(11) **EP 4 197 546 B1**
(45) Date of publication and mention of the grant of the patent: **28.01.2026**
(21) Application number: 21855620.7
(22) Date of filing: 13.08.2021
(51) Int. Cl.: A61K 35/28, A61K 35/12, A61K 9/00, A61K 35/50, A61P 9/10, A61K 35/545, A61K 35/51

(54) **USE OF PHARMACEUTICAL COMPOSITION FOR TREATING ISCHEMIA CONDITION OF TISSUE**
VERWENDUNG EINER PHARMAZEUTISCHEN ZUSAMMENSETZUNG ZUR BEHANDLUNG VON GEWEBEISCHÄMIE
UTILISATION D'UNE COMPOSITION PHARMACEUTIQUE POUR LE TRAITEMENT DE L'ISCÉMIE TISSULAIRE

(30) Priority: 14.08.2020 US 202063065728 P
(43) Date of publication of application: 21.06.2023
(73) Proprietor: ChironStem Inc., Rockville, MD 20850 (US)
(72) Inventor: SHYU, Woei-cherng, Taichung City, Taiwan 404 (TW); CHEN, Chien-lin, New Taipei City, Taiwan 220 (TW); JENG, Long-bin, Taichung City, Taiwan 404 (TW); TSAI, Chang-hai, Taichung City, Taiwan 404 (TW)
(74) Representative: D Young & Co LLP
(86) International application number: PCT/CN2021/112403
(87) International publication number: WO 2022/033570

(56) References cited:
- WO-A1-2007/024036
- CN-A- 101 820 890
- CN-A- 104 928 235
- CN-A- 105 560 284
- TW-A- 200 633 725
- TW-A- 201 704 466
- US-A1- 2009 246 179
- ANDR�IA VASCONCELOS-DOS-SANTOS ET AL: "Intravenous and intra-arterial administration of bone marrow mononuclear cells after focal cerebral ischemia: Is there a difference in biodistribution and efficacy?", STEM CELL RESEARCH, ELSEVIER, NL, vol. 9, no. 1, 16 February 2012 (2012-02-16), pages 1 - 8, XP028509830, ISSN: 1873-5061, [retrieved on 20120228], DOI: 10.1016/J.SCR.2012.02.002
- BANERJEE SOMA ET AL: "Intra-Arterial Immunoselected CD34+ Stem Cells for Acute Ischemic Stroke", vol. 3, no. 11, 8 August 2014 (2014-08-08), US, pages 1322 - 1330, XP093177414, ISSN: 2157-6564, Retrieved from the Internet <URL:https://api.wiley.com/onlinelibrary/tdm/v1/articles/10.5966%2Fsctm.2013-0178> DOI: 10.5966/sctm.2013-0178
- RODR�GUEZ-FRUTOS BERTA ET AL: "Stem Cell Therapy and Administration Routes After Stroke", TRANSLATIONAL STROKE RESEARCH, SPRINGER US, BOSTON, vol. 7, no. 5, 7 July 2016 (2016-07-07), pages 378 - 387, XP036052311, ISSN: 1868-4483, [retrieved on 20160707], DOI: 10.1007/S12975-016-0482-6
- KAMIYA N ET AL: "Intra-arterial transplantation of bone marrow mononuclear cells immediately after reperfusion decreases brain injury after focal ischemia in rats", LIFE SCIENCE, PERGAMON PRESS, OXFORD, GB, vol. 83, no. 11-12, 12 September 2008 (2008-09-12), pages 433 - 437, XP025350210, ISSN: 0024-3205, [retrieved on 20080808]
- ATSUHIKO TOYOSHIMA ET AL: "Intra-Arterial Transplantation of Allogeneic Mesenchymal Stem Cells Mounts Neuroprotective Effects in a Transient Ischemic Stroke Model in Rats: Analyses of Therapeutic Time Window and Its Mechanisms", PLOS ONE, vol. 10, no. 6, 15 June 2015 (2015-06-15), pages 1 - 17, XP055649767, DOI: 10.1371/journal.pone.0127302
- KASAHARA YUKIKO ET AL: "Transplantation of hematopoietic stem cells: intra-arterial versus intravenous administration impacts stroke outcomes in a murine model", TRANSLATIONAL RESEARCH, ELSEVIER, AMSTERDAM, NL, vol. 176, 18 April 2016 (2016-04-18), pages 69 - 80, XP029738566, ISSN: 1931-5244, DOI: 10.1016/J.TRSL.2016.04.003
- WANG, LI-XIN ET AL.: "Human Umbilical Cord Mesenchymal Stem Cell Transplantation by Different Pathways and Stem Cell Tracing by Bioluminescence Imaging in the Treatment of Ischemic Brain Injury", CHINESE JOURNAL OF TISSUE ENGINEERING RESEARCH, vol. 21, no. 33, 28 November 2017 (2017-11-28), pages 5407 - 5412, XP055900952

## Description

### BACKGROUND

### Technical Field

The present disclosure relates to a use of a pharmaceutical composition including a therapeutic cell. More particularly, the present disclosure relates to a use of a pharmaceutical composition including a therapeutic cell for treating an ischemia condition of a tissue.

### Description of Related Art

An ischemia condition is a condition in which blood supply to tissues is insufficient, resulting in a lack of oxygen and nutrients. Ischemia is generally caused by vascular problems, but ischemia can also be caused by vasoconstriction, thrombosis, or embolism. In addition to lack of oxygen and nutrients, ischemia can also lead to accumulation of metabolites that can damage tissues.

Ischemic heart disease occurs when the ischemia condition occurs in the heart. Ischemic heart disease is the leading cause of death worldwide. Many patients with heart failure secondary to acute myocardial ischemia are not suitable for invasive treatment, and there is no effective drug therapy, so new treatment methods are urgently needed. When the ischemia condition occurs in the brain, it causes ischemic stroke, which is also the leading cause of death worldwide. Ischemic stroke results in rapid loss of brain function due to abnormal blood supply to the brain and predisposes to neurological behavior impairment. In general, the conventional treatment of stroke mainly relies on drugs to prevent another stroke. In addition to the opportunity to use thrombolytic agents and intracranial arterial thrombectomy in the acute stage of ischemic stroke, other drug treatments are mainly to reduce the risk of re-stroke. However, these drugs have limited effect on the recovery of damaged brain tissue and neurological function after stroke.

Currently in regenerative medicine, therapeutic cells such as stem cell have been found to be used as novel treatments for the ischemic heart disease and the ischemic stroke due to their pluripotency and self-renewal capacity. However, poor engraftment has been observed in the treatment of ischemic heart disease with the therapeutic cells, possibly due to poor viability of the implanted therapeutic cells in infarcted cardiac tissue. Only 1% of viable cells are present 4 days after the transplantation, resulting in little improvement in cardiac function when the therapeutic cells are implanted into the infarct site. Therefore, method to increase the survival rate of the therapeutic cells is paramount important in treatment. In the treatment of ischemic stroke with the therapeutic cells, when the therapeutic cells are implanted intravenously, most of the therapeutic cells remain in lung and liver of the subject. Therefore, how to increase the number of the therapeutic cells in and around the infarcted area to improve the therapeutic effect is also an urgent problem to be solved.RODRÍGUEZ-FRUTOS BERTA ET AL: "Stem Cell Therapy and Administration Routes After Stroke",TRANSLATIONAL STROKE RESEARCH, SPRINGER US, BOSTON, vol. 7, no. 5, 7 July 2016 (2016-07-07), pages 378-387 and ANDRÉIA VASCONCELOS-DOS-SANTOS ET AL: "Intravenous and intra-arterial administration of bone marrow mononuclear cells after focal cerebral ischemia: Is there a difference in biodistribution and efficacy?",STEM CELL RESEARCH, ELSEVIER, NL, vol. 9, no. 1, 16 February 2012 (2012-02-16), pages 1-8 discuss administration routes for stem cell therapy.

### SUMMARY

According to one aspect of the present disclosure is to provide a pharmaceutical composition for use in treatment of an ischemia condition of a tissue, wherein the pharmaceutical composition includes a therapeutic cell, which therapeutic cell is a mesenchymal stem cell, a first dose of the pharmaceutical composition is administered by an intra-arterial injection, and then a second dose of the pharmaceutical composition is administered by an intra-venous injection, wherein an administration time interval between the first dose of the pharmaceutical composition and the second dose of the pharmaceutical composition is 2-4 hours.

According to the pharmaceutical composition for use, wherein the mesenchymal stem cell can be an umbilical cord mesenchymal stem cell, a bone marrow mesenchymal stem cell or an adipose mesenchymal stem cell.

According to the pharmaceutical composition for use, wherein the first dose of the pharmaceutical composition can be administered 24 hours after the ischemia condition of the tissue occurs.

According to the pharmaceutical composition for use, wherein the tissue can be a brain. Preferably, the ischemia condition can be an ischemic stroke.

According to the pharmaceutical composition for use, wherein the first dose of the pharmaceutical composition can include at least 1×10⁵ of the therapeutic cells, and the second dose of the pharmaceutical composition can include at least 1×10⁶ of the therapeutic cells.

According to the pharmaceutical composition for use, wherein the tissue can be a heart. Preferably, the ischemia condition can be a myocardial infarction.

According to the pharmaceutical composition for use, wherein the first dose of the pharmaceutical composition can include at least 0.5×10⁶ of the therapeutic cells, and the second dose of the pharmaceutical composition can include at least 1×10⁶ of the therapeutic cells.

### BRIEF DESCRIPTION OF THE DRAWINGS

The present disclosure can be more fully understood by reading the following detailed description of the embodiment, with reference made to the accompanying drawings as follows:
Fig. 1, Fig. 2A and Fig. 2B show the analysis results of the cerebral infarction in stroke rats after administration of umbilical cord mesenchymal stem cell (UMSC) by different administration routes;
Fig. 2C and Fig. 2D show the analysis results of the cerebral infarction in the stroke rats after administration of adipose mesenchymal stem cell (ADSC) by different administration routes;
Fig. 2E and Fig. 2F show the analysis results of the cerebral infarction in the stroke rats after administration of bone marrow mesenchymal stem cell (BMSC) by different administration routes;
Fig. 3A, Fig. 3B and Fig. 3C show the analysis results of the improvement of the neurological behavior in the stroke rats after administration of UMSC by different administration routes;
Fig. 4A, Fig. 4B and Fig. 4C show the analysis results of the improvement of the neurological behavior in the stroke rats after administration of ADSC by different administration routes;
Fig. 5A, Fig. 5B and Fig. 5C show the analysis results of improvement of the neurological behavior in the stroke rats after administration of BMSC by different administration routes;
Fig. 6A, Fig. 6B and Fig. 6C show the analysis results of the cardiac infarction in acute myocardial infarction (AMI) rats after administration of UMSC by different administration routes;
Fig. 7A and Fig. 7B show the analysis results of the cardiac infarction in the AMI rats after administration of ADSC by different administration routes;
Fig. 8A and Fig. 8B show the analysis results of the cardiac infarction in the AMI rats after administration of BMSC by different administration routes;
Fig. 9A, Fig. 9B, Fig. 9C and Fig. 9D show the analysis results of cardiac inflammation in the AMI rats after administration of UMSC, ADSC or BMSC by different administration routes; and
Fig. 10A, Fig. 10B, Fig. 10C and Fig. 10D show the analysis results of cardiac fibrosis in the AMI rats after administration of UMSC, ADSC or BMSC by different administration routes.

### DETAILED DESCRIPTION

The present disclosure provides a novel transplantation strategy of a therapeutic cell to administer a pharmaceutical composition including the therapeutic cell by an intra-arterial injection combined with an intra-venous injection to a subject having an ischemia condition of a tissue, which therapeutic cell is a mesenchymal stem cell, wherein an administration time interval between the first dose of the pharmaceutical composition and the second dose of the pharmaceutical composition is 2-4 hours.

Furthermore, the therapeutic cell can be a cell that can reconfigure and repair the tissue with the ischemia condition, improve functional damage caused by the ischemia condition of the tissue, or treat the ischemia condition of the tissue. Preferably, the therapeutic cell can be an umbilical cord mesenchymal stem cell, a bone marrow mesenchymal stem cell or an adipose mesenchymal stem cell. An administration route of the pharmaceutical composition can be to administer a first dose of the pharmaceutical composition by the intra-arterial injection, and then administer a second dose of the pharmaceutical composition by the intra-venous injection. When the tissue with the ischemia condition is a brain, the first dose of the pharmaceutical composition can include at least 1×10⁵ of the therapeutic cells, and the second dose of the pharmaceutical composition can include at least 1×10⁶ of the therapeutic cells. When the tissue with the ischemia condition is a heart, the first dose of the pharmaceutical composition can include at least 0.5×10⁶ of the therapeutic cells, and the second dose of the pharmaceutical composition can include at least 1×10⁶ of the therapeutic cells. Preferably, the first dose of the pharmaceutical composition can be administered 24 hours after the ischemia condition of the tissue occurs.

The novel transplantation strategy of the therapeutic cell of the present disclosure can be proved to be a safe strategy by animal experiments in stroke rats and acute myocardial infarction rats. Compared with the group in which the pharmaceutical composition including the therapeutic cell is administered by the intra-arterial injection or the intra-venous injection alone, the pharmaceutical composition including the therapeutic cell is administered by the combination of the intra-arterial injection and the intra-venous injection has a synergistic effect and can significantly improve the cardiac function after ischemia and the neurological function after cerebral ischemia, so it can be used to treat the ischemia condition of the tissue, in which the tissue can be the brain or the heart. When the tissue is the brain, the ischemia condition can be an ischemic stroke; when the tissue is the heart, the ischemia condition can be a myocardial infarction.

Unless otherwise noted, all terms, symbols or other scientific terms or terms used in the present disclosure have the meanings that are commonly understood by person having ordinary skill in the art. In some cases, terms with conventional meanings are defined herein for clarity and/or immediate reference, and the definitions incorporated herein should be construed as not necessarily substantial different from the conventional meanings in the art. Many of the techniques and procedures described or referenced herein are well known and routinely used by those skilled in the art. Where appropriate, unless otherwise stated, procedures for the use of commercially available kits and reagents are generally performed according to instructions and/or parameters defined by the manufacturer.

"Treating," "treat," or "treatment" refers to administering the pharmaceutical composition of the present disclosure to a subject in need, such as a patient with an ischemic heart disease or the ischemic stroke.

"Ameliorating" refers to reducing, inhibiting, attenuating, decreasing, halting or stabilizing the development or progression of a disease or its symptoms.

"Intra-arterial injection (IA)" refers to an administration route in which liquid substances such as blood, medicinal solution, nutrient solution, and cellular fluid are directly injected into the artery.

"Intra-venous injection (IV)" refers to an administration route in which liquid substances such as blood, medicinal solution, nutrient solution, and cellular fluid are directly injected into the vein.

The following specific examples are used to further illustrate the present disclosure, in order to benefit the person having ordinary skill in the art, and can fully utilize and practice the present disclosure without excessive interpretation. These examples should not be regarded as limiting the scope of the present disclosure, but is used to illustrate how to implement the materials and methods of the present disclosure.

### [Test Examples]

The test examples investigate the effect of the transplantation strategy of the therapeutic cell of the present disclosure, using the pharmaceutical composition including the therapeutic cell to treat the ischemia condition of the tissue. The therapeutic cells used in the test examples are the umbilical cord mesenchymal stem cell (hereinafter referred to as UMSC), the adipose mesenchymal stem cell (hereinafter referred to as ADSC) and the bone marrow mesenchymal stem cell (hereinafter referred to as BMSC), are all purchased from American Type Culture Collection (ATCC), and their ATCC numbers are UMSC (ATCC PCS 500-010), ADSC (ATCC PCS 500-011) and BMSC (ATCC PCS 500-012). Groups in which the pharmaceutical composition including the therapeutic cell is administered by the intra-arterial injection (IA) or the intra-venous injection (IV) alone are also included, and groups in which the pharmaceutical composition including the therapeutic cell is administered by the intra-arterial injection combined with the intra-venous injection (IA-IV). Therefore, the groups in the following test examples include: the pharmaceutical composition including UMSC administered by the intra-arterial injection alone (hereinafter referred to as IA-UMSC), the pharmaceutical composition including UMSC administered by the intra-venous injection alone (hereinafter referred to as IV-UMSC), the pharmaceutical composition including UMSC administered by the intra-arterial injection combined with the intra-venous injection (hereinafter referred to as IA-IV-UMSC), the pharmaceutical composition including ADSC administered by the intra-arterial injection alone (hereinafter referred to as IA-ADSC), the pharmaceutical composition including ADSC administered by the intra-venous injection alone (hereinafter referred to as IV-ADSC), the pharmaceutical composition including ADSC administered by the intra-arterial injection combined with the intra-venous injection (hereinafter referred to as IA-IV-ADSC), the pharmaceutical composition including BMSC administered by the intra-arterial injection alone (hereinafter referred to as IA-BMSC), the pharmaceutical composition including BMSC administered by the intra-venous injection alone (hereinafter referred to as IV-BMSC), and the pharmaceutical composition including BMSC administered by the intra-arterial injection combined with the intra-venous injection (hereinafter referred to as IA-IV-BMSC).

### I. Use for treating the ischemia condition of the brain

In this test example, a stroke rat model is used to evaluate whether the transplantation strategy of the therapeutic cell of the present disclosure can reduce the volume of cerebral infarction in the stroke rats. Rats before and after the stroke are measured by three modalities of neurological deficits to evaluate the neurological recovery.

An ischemia-reperfusion model is used to simulate transient focal cerebral ischemia in rats with three-vessel ligation. Test animals are male SD (Sprague-Dawley) rats weighing 250-300 g. All surgical procedures, animals' experimental protocols and methods are performed in accordance with the "Guidelines for the Care and Use of Laboratory Animals" and approved by the Institutional Committee for Animal and Clinical Research of China Medical University. To establish the ischemia-reperfusion model, the rats are anesthetized by intraperitoneal injection of 0.4 g/kg chloral hydrate, then the bilateral common carotid arteries (CCA) are clamped with non-traumatic arterial clips, and the right middle cerebral artery (MCA) is ligated with 10-0 nylon suture to induce the focal cerebral ischemia. After 90 minutes ischemia, the suture on the MCA and the arterial clips on CCAs are removed to allow reperfusion. Core body temperature of each of the rats is monitored by a thermistor probe and maintained at 37°C with a heating pad during anesthesia. After recovery from anesthesia, the body temperature of the rats is maintained at 37°C with a heat lamp.

After the establishment of the stroke rat model, the stroke rats are administered the pharmaceutical composition including UMSC, ADSC or BMSC in different administration routes, and the test example also includes a group only treated with phosphate buffered saline (PBS) as a control group. In the groups administered by the intra-arterial injection alone (IA-UMSC, IA-ADSC or IA-BMSC), 24 hours after the stroke, the stroke rats are anesthetized by intraperitoneal injection of 0.4 g/kg of chloral hydrate. The ipsilateral common carotid artery of each of the stroke rats is again exposed, the external carotid artery is ligated with 6-0 silk, the superior thyroid and pterygopalatine arteries are coagulated, and UMSC, ADSC or BMSC (the amount of cells is 1×10⁵) in 300 µL PBS are injected into the internal carotid artery using a 30-G angio-catheter under stop-flow technique, which is performed by blocking the proximal blood flow during the cells injections procedure through the angio-catheter for 3 minutes. In the groups administered by the intra-venous injection (IV-UMSC, IV-ADSC or IV-BMSC), 2 hours after the stroke, the stroke rats are anesthetized by intraperitoneal injection of 0.4 g/kg of chloral hydrate, and then UMSC, ADSC or BMSC (the amount of cells is 1×10⁶) in 1 mL PBS are injected into the femoral vein using a 27-G angio-catheter. In the groups administered by the intra-arterial injection combined with the intra-venous injection (IA-IV-UMSC, IA-IV-ADSC or IA-IV-BMSC), 24 hours after the stroke, UMSC, ADSC or BMSC (the amount of cells is 1×10⁵) in 300 µL PBS are injected at 24 hours after the stroke into the internal carotid artery, and then UMSC, ADSC or BMSC (the amount of cells is 1×10⁶) in 1 mL PBS are injected into the femoral vein at 26 hours after the stroke. Because of the immunosuppressive characteristics of the mesenchymal stem cells, test rats do not receive any immunosuppressive medication.

### 1.1 Reduction of volume of cerebral infarct in the stroke rats

This test example will further explore whether the transplantation strategy of the therapeutic cell of the present disclosure can reduce the volume of cerebral infarction in the stroke rats. The stroke rats are sacrificed 3 days after a transplantation of UMSC, ADSC or BMSC, and the brain tissues are stained with triphenyltetrazolium chloride (TTC). After staining with triphenyltetrazolium chloride, the non-infarcted area is brick red, and the infarcted area is yellowish white, which can be used to observe the size of the infarcted area in the brain of the stroke rats to determine whether the transplantation of UMSC, ADSC or BMSC can protect the stroke rats from damage caused by ischemia.

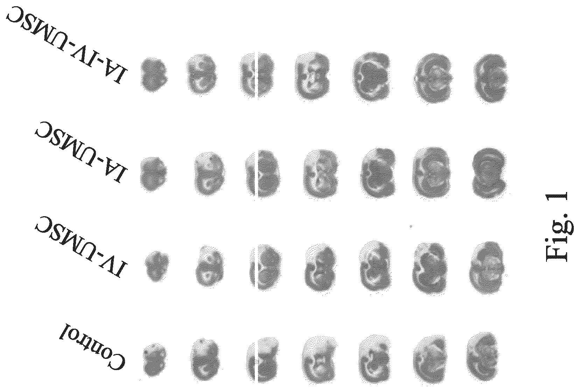

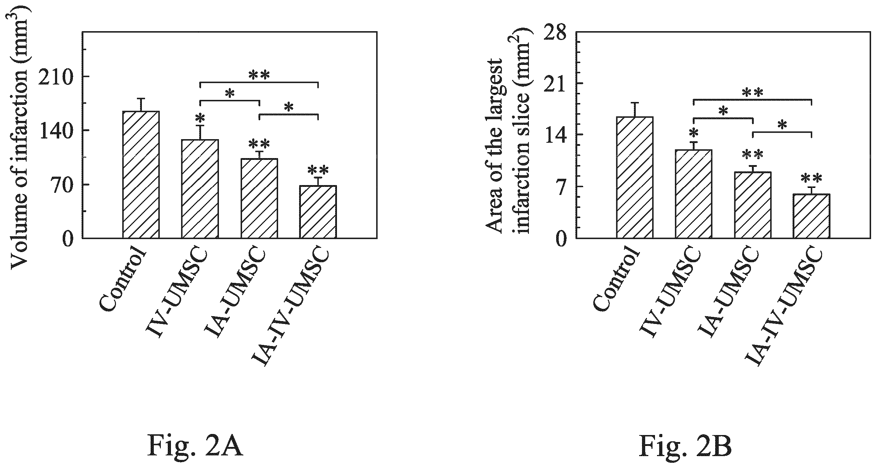

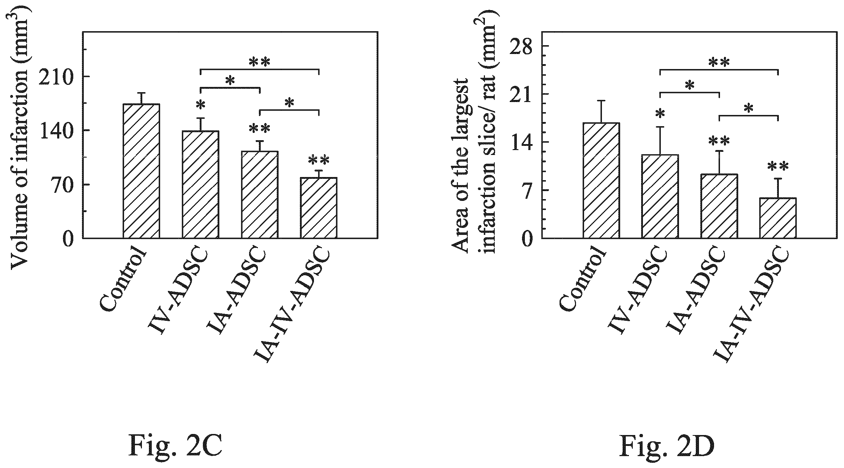

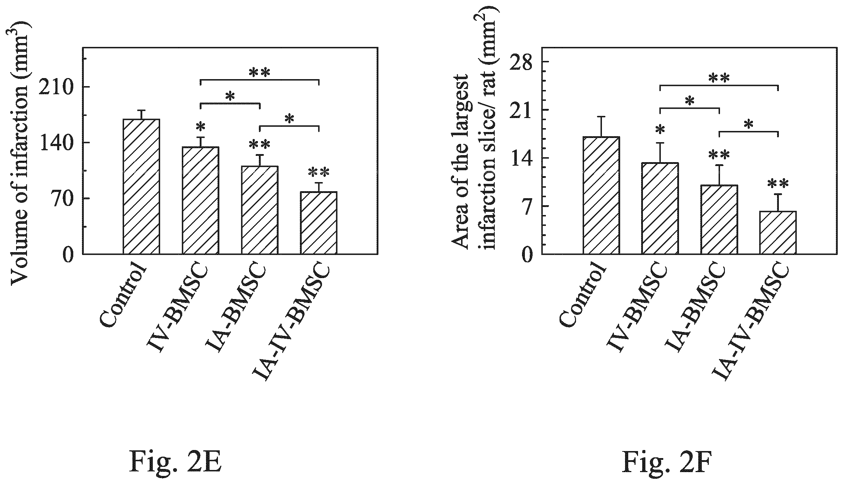

Reference is made to Fig. 1 to Fig. 2F, wherein Fig. 1, Fig. 2A and Fig. 2B show the analysis results of the cerebral infarction in the stroke rats of the control group, IA-UMSC, IV-UMSC and IA-IV-UMSC; Fig. 2C and Fig. 2D show the analysis results of the cerebral infarction in the stroke rats of the control group, IA-ADSC, IV-ADSC and IA-IV-ADSC; Fig. 2E and Fig. 2F show the analysis results of the cerebral infarction in the stroke rats of the control group, IA-BMSC, IV-BMSC and IA-IV-BMSC. Data are expressed as mean ± SEM, * represents p< 0.05 and ** represents p< 0.01.

In Fig. 1, the stroke rats of IA-IV-UMSC show mild infarction 3 days after the cerebral ischemia. In Fig. 2A and Fig. 2B, quantitative measurement of the infarction volume reveals that infarct volume is significantly reduced in IA-IV-UMSC compared to IV-UMSC, IA-UMSC, and the control group. Consistently, the area of the largest infarction is smaller in IA-IV-UMSC than the largest infarction in IV-UMSC, IA-UMSC, and the control group. As shown in the results in Fig. 2C and Fig. 2D, the infarct volume of IA-IV-ADSC is significantly reduced compared to IV-ADSC, IA-ADSC, and the control group, and the area of the largest infarction of IA-IV-ADSC is also smaller than that of IV-ADSC, IA-ADSC, and the control group. As shown in the results in Fig. 2E and Fig. 2F, the infarct volume of IA-IV-BMSC is significantly reduced compared to IV-BMSC, IA-BMSC, and the control group, and the area of the largest infarction of IA-IV-BMSC is also smaller than IV-BMSC, IA-BMSC and the control group. The results indicate that the transplantation strategy of the therapeutic cell of the present disclosure can significantly reduce the infarct volume in the brain of the stroke rats.

### 1.2 Improvement of neurological behavior in the stroke rats

Behavioral assessments are performed 5 days before the cerebral ischemia, and 1, 7, 14 and 28 days after the transplantation. The three models of neurological deficits are to assess body asymmetry and locomotor activity of the rat, respectively. An elevated body swing test is used to assess the body asymmetry of the rats. Initially, the rats are suspended by their tail 10 cm above the cage floor, and lateral body movements are recorded. Specifically, the frequency with which the initial head swing contra-lateral to the ischemic side is counted in 20 consecutive tests and is normalized to the baseline score. The locomotor activity of the rats is measured for about 2 hours using VersaMax Animal Activity Monitoring System (Accuscan Instruments). The VersaMax Animal Activity Monitoring System contains 16 horizontal infrared sensors and 8 vertical infrared sensors, in which the vertical sensors are situated 10 cm above the chamber floor and the locomotor activity is quantified by a number of a beam broken by the rat's movement in the chamber. Three vertical-movement parameters are measured: vertical activity, vertical time, and number of vertical movements.

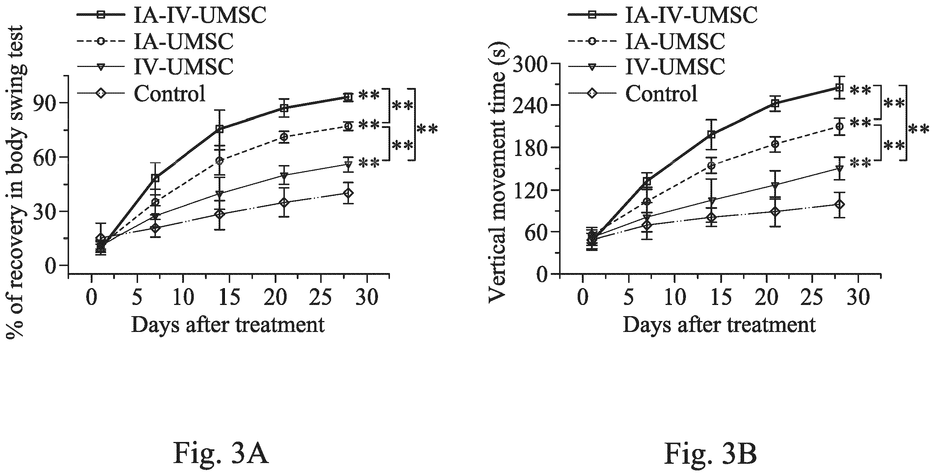

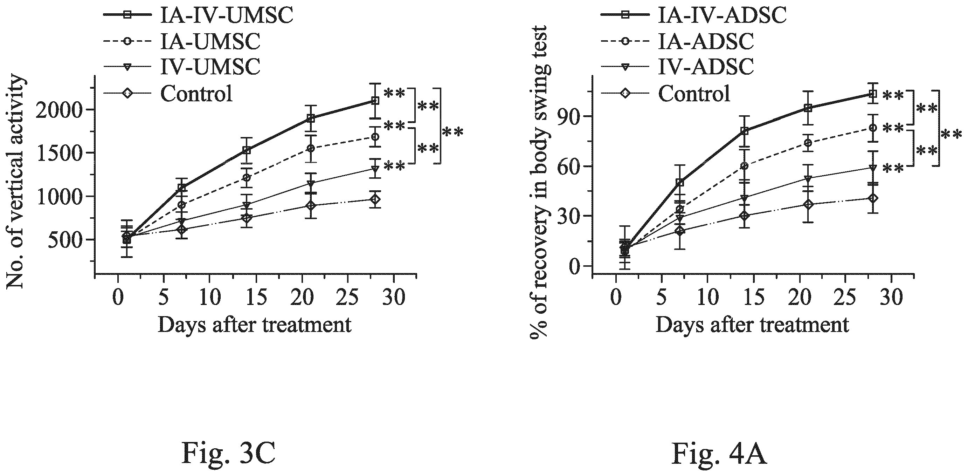

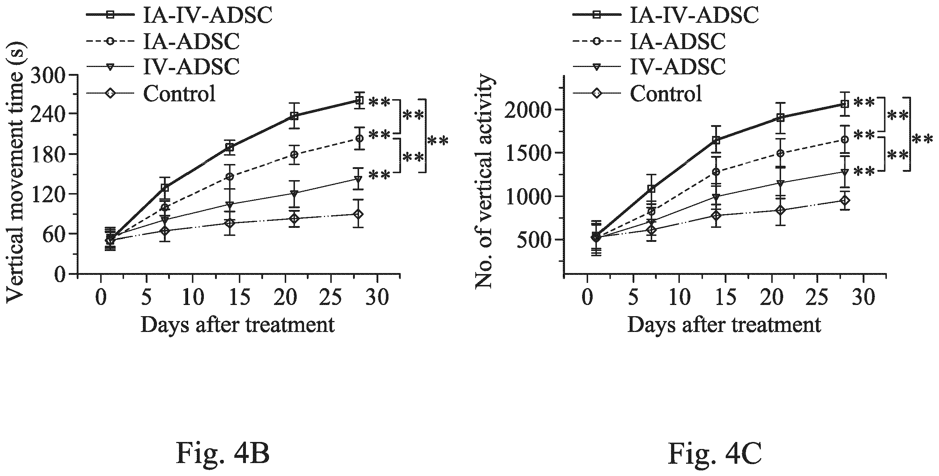

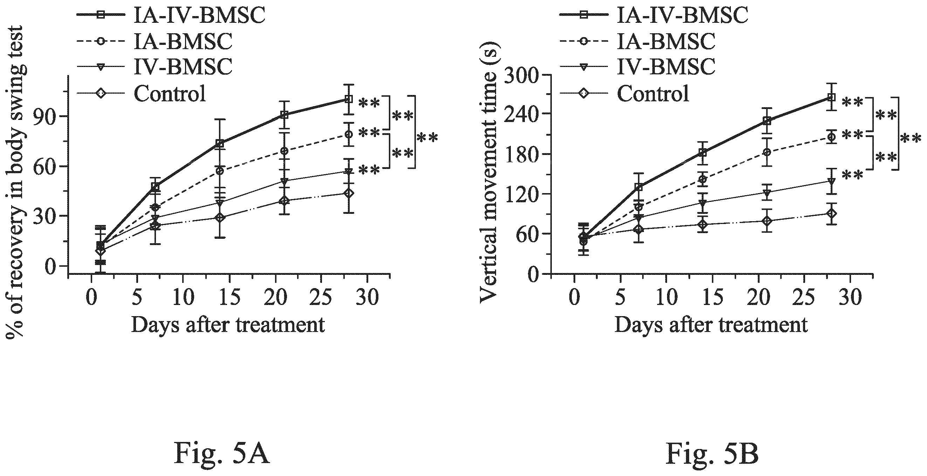

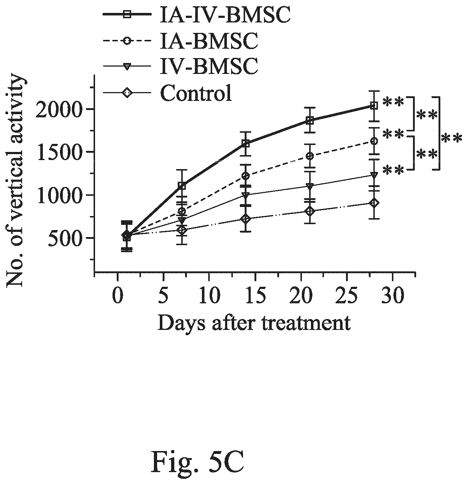

Reference is made to Fig. 3A to Fig. 5C, wherein Fig. 3A, Fig. 3B and Fig. 3C show the analysis results of the improvement of the neurological behavior in the stroke rats of the control group, IA-UMSC, IV-UMSC and IA-IV-UMSC; Fig. 4A, Fig. 4B and Fig. 4C show the analysis results of the improvement of the neurological behavior in the stroke rats of the control group, IA-ADSC, IV-ADSC and IA-IV-ADSC; Fig. 5A, Fig. 5B and Fig. 5C show the analysis results of the improvement of neurological behavior in the stroke rats of the control group, IA-BMSC, IV-BMSC and IA-IV-BMSC. Data are expressed as mean ± SEM, and ** represents p< 0.01.

The results in Fig. 3A, Fig. 3B and Fig. 3C show that the transplantation mode of IA-IV-UMSC significantly improves the neurological behavior of the stroke rats, regardless of the measurement of the body asymmetry or the locomotor activity. Better recovery is found in IA-IV-UMSC than IA-UMSC, IV-UMSC and the control group in the body asymmetry assay. Significant improvements of neurological deficit in the locomotor activity are also observed in the IA-IV-UMSC compared to IA-UMSC, IV-UMSC and the control group. The results indicate that the transplantation mode of IA-IV-UMSC can make UMSC have superior neuroregenerative potential.

The results in Figs. 4A, 4B, and 4C show that better recovery is found in IA-IV-ADSC than IA-ADSC, IV-ADSC, and the control group in the body asymmetry assay. Significant improvements of neurological deficit in the locomotor activity are also observed in the IA-IV-ADSC compared to IA-ADSC, IV-ADSC and the control group. The results indicate that the transplantation mode of IA-IV-ADSC can significantly improve the neurological behavior in the stroke rats. The results in Figs. 5A, 5B and 5C show that better recovery is found in IA-IV-BMSC than IA-BMSC, IV-BMSC, and the control group in the body asymmetry assay. Significant improvements of neurological deficit in the locomotor activity are also observed in the IA-IV-BMSC compared to IA-BMSC, IV-BMSC and the control group. The results indicate that the transplantation mode of IA-IV-BMSC can significantly improve the neurological behavior in the stroke rats.

### II. Use for treating the ischemia condition of the heart

In this test example, an acute myocardial infraction (AMI) rat model is used to evaluate whether the transplantation strategy of the therapeutic cell of the present disclosure can improve myocardial function recovery after myocardial infarction.

The rats are subjected to AMI by ligation of left anterior descending (LAD) coronary artery to simulate transient cardiac ischemia symptoms in the rats. The test animals are male SD rats weighing 250-300 g. In brief, after induction of anaesthesia with 2% isoflurane (in 100% oxygen), the rats receive artificial ventilation using a respirator (SN-480-7) with a tidal volume of 1mL/100g and respiratory rate 80/min. A left thoracotomy is performed in the 4-5th intercostal space using a rib retractor (MY-9454S), and the left lung is deflated using a small piece of gauze soaked in saline. The pericardium is then removed and a 6-0-polyethylene suture needle with thread (Ethicon) is used to ligature the LAD coronary artery. When ligation area becomes white and T-wave of an electrocardiogram great rise, lungs are then re-inflated before the thorax is closed, and the AMI rat model is completed.

After the establishment of the AMI rat model, the AMI rats are administered the pharmaceutical composition including UMSC, ADSC or BMSC in different administration routes, and the test example also includes a group only treated with PBS as a control group. In the groups administered by the intra-arterial injection alone (IA-UMSC, IA-ADSC or IA-BMSC), 24 hours after the AMI, the AMI rats are anesthetized by intraperitoneal injection of 0.4 g/kg of chloral hydrate. The left common carotid artery of each of the AMI rats is again exposed, the external carotid artery is ligated with 6-0 silk, the superior thyroid and pterygopalatine arteries are coagulated, and UMSC, ADSC or BMSC (the amount of cells is 0.5×10⁶) in 500 µL PBS are injected into the internal carotid artery using a 30-G angio-catheter under stop-flow technique, which is performed by blocking the proximal blood flow during the cells injections procedure through the angio-catheter for 3 minutes. In the groups administered by the intra-venous injection (IV-UMSC, IV-ADSC or IV-BMSC), 2 hours after the AMI, the AMI rats are anesthetized by intraperitoneal injection of 0.4 g/kg of chloral hydrate, and then UMSC, ADSC or BMSC (the amount of cells is 1×10⁶) in 1 mL PBS are injected into the femoral vein using the 27-G angio-catheter. In the groups administered by the intra-arterial injection combined with the intra-venous injection (IA-IV-UMSC, IA-IV-ADSC or IA-IV-BMSC), 24 hours after the AMI, UMSC, ADSC or BMSC (the amount of cells is 0.5×10⁶) in 500 µL PBS are injected at 24 hours after the AMI into the internal carotid artery, and then UMSC, ADSC or BMSC (the amount of cells is 1×10⁶) in 1 mL PBS are injected into the femoral vein at 26 hours after the AMI.

### 2.1 Reduction of volume of cardiac infarct in the AMI rats

This test example will further explore whether the transplantation strategy of the therapeutic cell of the present disclosure can reduce the volume of cardiac infarct in the AMI rats. The AMI rats are sacrificed 28 days after the transplantation of UMSC, ADSC or BMSC, and the heart tissue sections are soaked in triphenyltetrazolium chloride, and then soaked in dehydrogenase, wherein the necrotic area is stained red-blue, and the area without the myocardial infarction is stained deep red. Stained sections at various levels (apex, middle and base of the left ventricle) along the long axis are analyzed using ImageJ software (NIH) to calculate infarct area and infarct wall thickness.

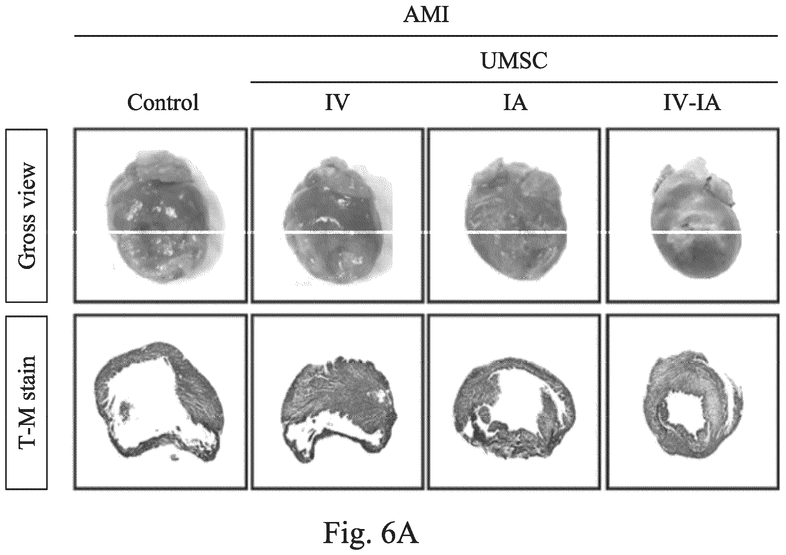

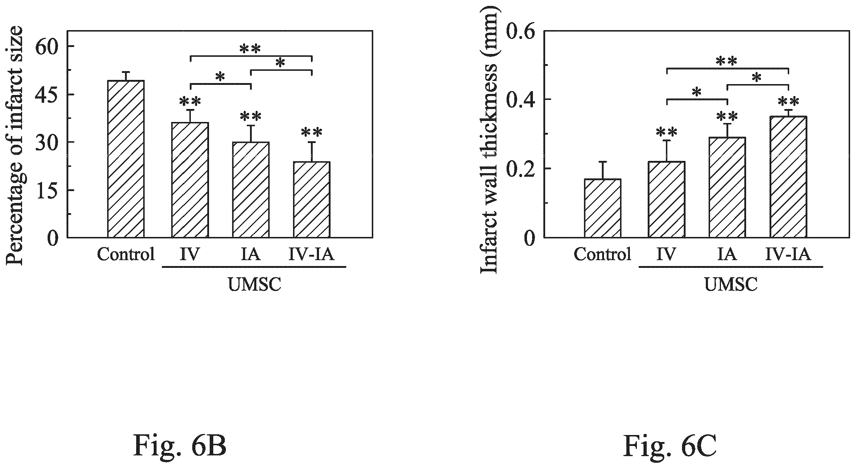

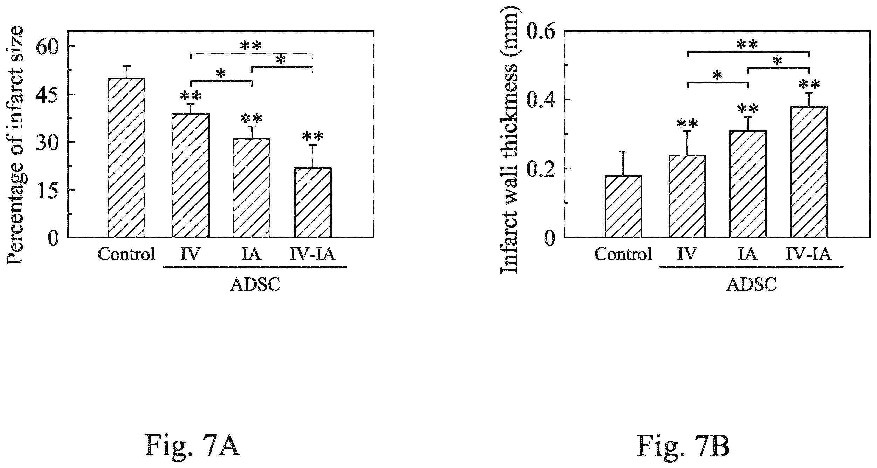

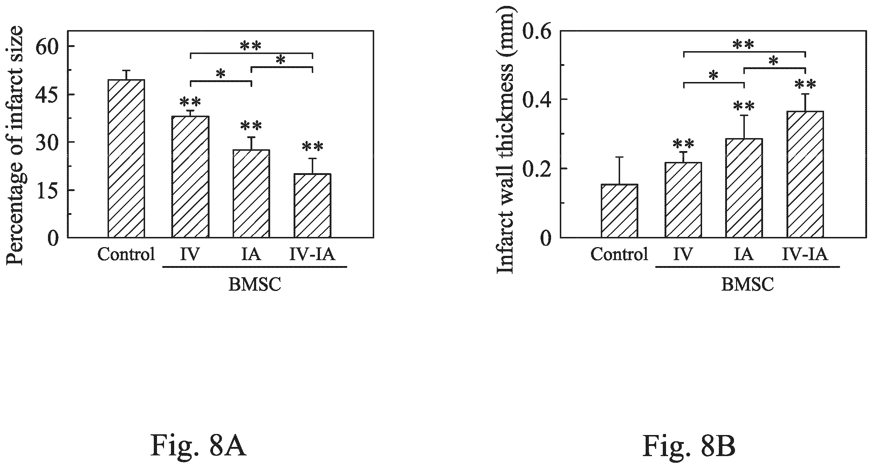

Reference is made to Fig. 6A to Fig. 8B, wherein Fig. 6A, Fig. 6B and Fig. 6C show the analysis results of the cardiac infarction in the AMI rats of the control group, IA-UMSC, IV-UMSC and IA-IV-UMSC; Fig. 7A and Fig. 7B show the analysis results of the cardiac infarction in the AMI rats of the control group, IA-ADSC, IV-ADSC and IA-IV-ADSC; Fig. 8A and Fig. 8B show the analysis results of the cardiac infarction in the AMI rats of the control group, IA-BMSC, IV-BMSC and IA-IV-BMSC. Data are expressed as mean ± SEM, * represents p< 0.05 and ** represents p< 0.01.

The results in Fig. 6A show that the AMI rats of IA-IV-UMSC presented mild infarction 28 days after the AMI. In Fig. 6B and Fig. 6C, quantitative measurement of the infarction volume reveals that the infarct volume is significantly reduced in IA-IV-UMSC compared to IV-UMSC, IA-UMSC, and the control group. The results of the infarct wall thickness are consistent with the results of infarct volume changes; the infarct wall thickness of IA-IV-UMSC is increased compared with IV-UMSC, IA-UMSC and the control group. As shown in the results in Fig. 7A and Fig. 7B, the infarct volume of IA-IV-ADSC is significantly reduced compared to IV-ADSC, IA-ADSC, and the control group, and the infarct wall thickness of IA-IV-ADSC is also greater than that of IV-ADSC, IA-ADSC, and the control group. As shown in the results in Fig. 8A and Fig. 8B, the infarct volume of IA-IV-BMSC is significantly reduced compared to IV-BMSC, IA-BMSC, and the control group, and the infarct wall thickness of IA-IV-BMSC is also greater than IV-BMSC, IA-BMSC and the control group. The results indicate that the transplantation strategy of the therapeutic cell of the present disclosure can enable the therapeutic cell to play an important role in rescue of cardiac ischemic injury.

### 2.2 Anti-inflammatory effects on ischemic cardiac tissue

This test example will further explore whether the transplantation strategy of the therapeutic cell of the present disclosure can inhibit the inflammatory response after the myocardial infarction. The AMI rats are sacrificed 3 days after the transplantation of UMSC, ADSC or BMSC, and the heart tissue sections are stained with H&E staining to detect the degree of inflammation and analyzed by light microscopy (Nikon, E600). The inflammatory conditions of the heart tissue are categorized into 0-5 grades, where grade 0 represents no inflammation, grade 1 represents inflammation in less than 5% of the heart tissue sections, grade 2 represents inflammation in 6% to 10% of the heart tissue sections, grade 3 represents inflammation in 11% to 30% of the heart tissue sections, grade 4 represents inflammation in 31% to 50% of the heart tissue sections, and grade 5 represents inflammation in greater than 50% of the heart tissue sections. The results of different groups are classified and counted according to the above criteria.

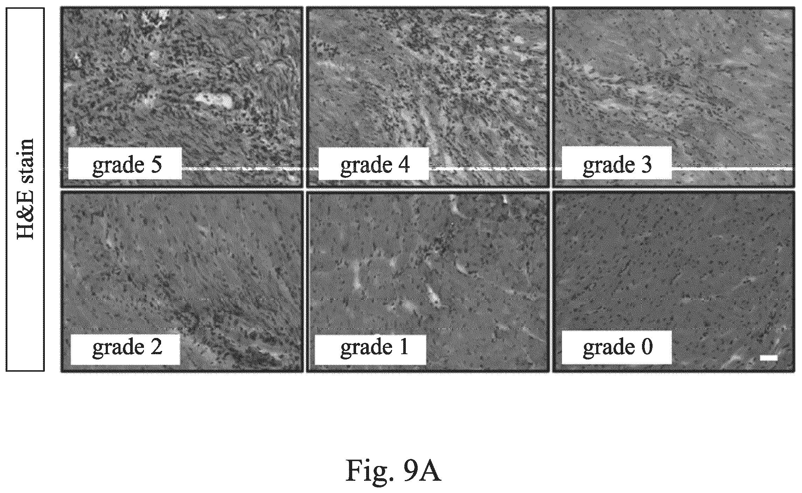

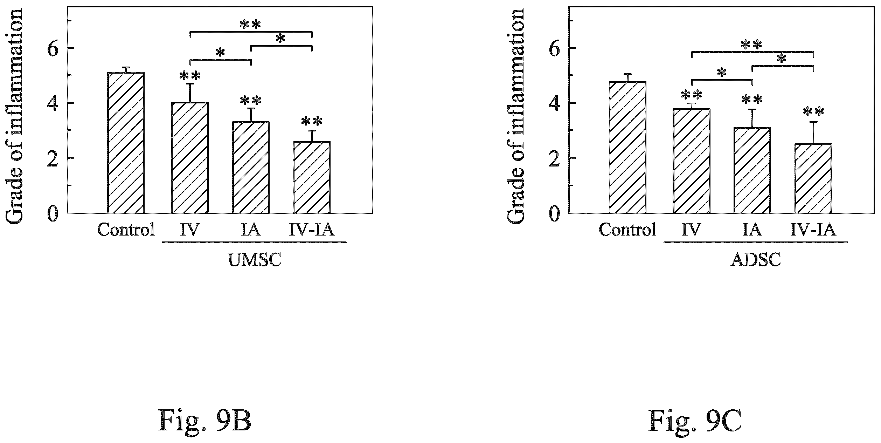

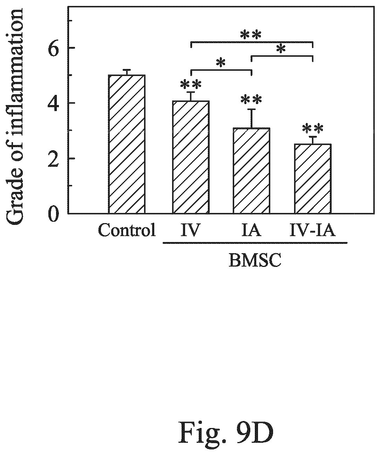

Reference is made to Fig. 9A, Fig. 9B, Fig. 9C and Fig. 9D, which show the analysis results of cardiac inflammation in the AMI rats after administration of UMSC, ADSC or BMSC by different administration routes. Fig. 9B shows the analysis results of groups of IA-UMSC, IV-UMSC and IA-IV-UMSC, Fig. 9C shows the analysis results of groups of IA-ADSC, IV-ADSC and IA-IV-ADSC, and Fig. 9D shows the analysis results of groups of IA-BMSC, IV-BMSC and IA-IV-BMSC. Data are expressed as mean ± SEM, * represents p< 0.05 and ** represents p< 0.01.

The results in Fig. 9A and Fig. 9B show that significant reduction of inflammation is observed in IA-IV-UMSC compared to IV-UMSC, IA-UMSC and the control group. As shown in the results in Fig. 9C, significant reduction of inflammation is observed in IA-IV-ADSC compared to IV-ADSC, IA-ADSC and the control group. The results in Fig. 9D also show that significant reduction of inflammation is observed in IA-IV-BMSC compared to IV-BMSC, IA-BMSC and the control group. The results indicate that the transplantation strategy of the therapeutic cell of the present disclosure can improve the ability of the therapeutic cell to inhibit the inflammatory response after the myocardial infarction.

### 2.3 Reduction of fibrosis caused by myocardial infarction

Myocardial fibers are different from muscle fibers, the myocardial fibers can work long hours to pump blood into every part of a body. The myocardial fibers will produce irreversible necrosis after the myocardial infarction. The necrosis part is replaced with fibrous tissue, and then fibrosis is generated in a few weeks. This test example will further explore whether the transplantation strategy of the therapeutic cell of the present disclosure can reduce myocardial infarction-induced fibrosis. The AMI rats are sacrificed 28 days after the transplantation of UMSC, ADSC or BMSC, and the heart tissue sections are stained with Masson's trichrome staining to observe the fibrosis of rat heart tissue, in which collagen fibers are stained blue, and muscle fibers are stained red. The heart tissue sections are analyzed by light microscopy (Nikon, E600). The fibrosis conditions of the heart tissue are categorized into 0-5 grades, where grade 0 represents no fibrosis, grade 1 represents fibrosis in less than 5% of the heart tissue sections, grade 2 represents fibrosis in 6% to 10% of the heart tissue sections, grade 3 represents fibrosis in 11% to 30% of the heart tissue sections, grade 4 represents fibrosis in 31% to 50% of the heart tissue sections, and grade 5 represents fibrosis in greater than 50% of the heart tissue sections. The results of different groups are classified and counted according to the above criteria.

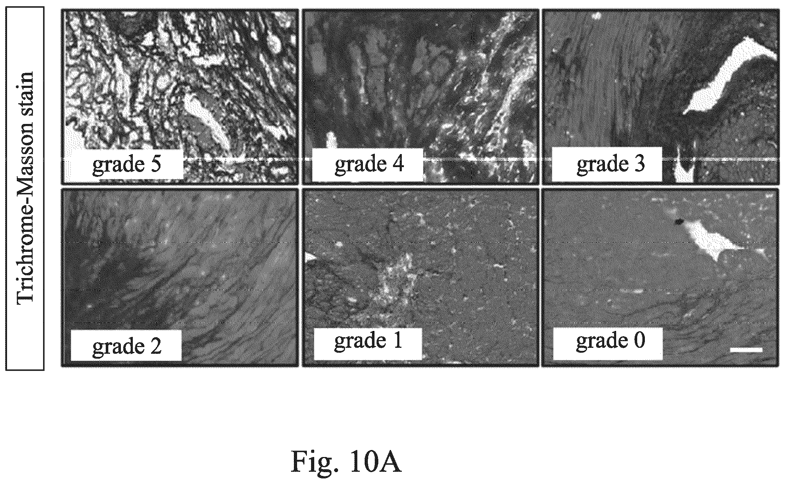

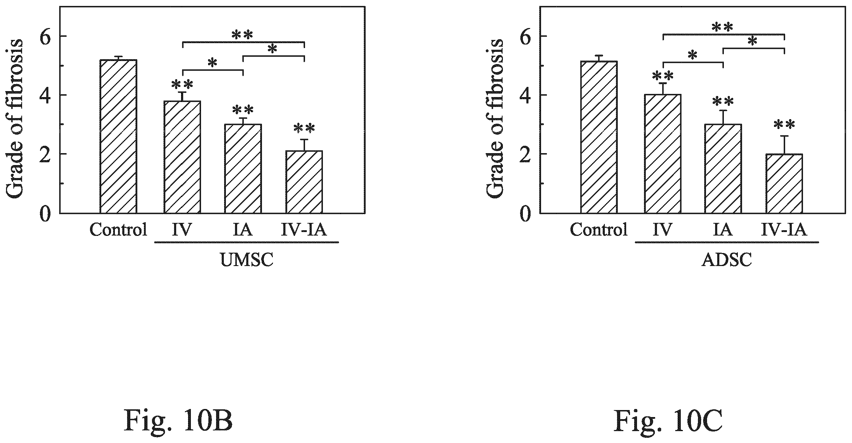

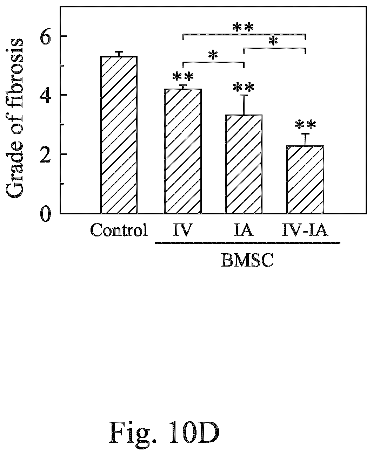

Reference is made to Fig. 10A, Fig. 10B, Fig. 10C and Fig. 10D, which show the analysis results of the cardiac fibrosis in the AMI rats after administration of UMSC, ADSC or BMSC by different administration routes. Fig. 10B shows the analysis results of IA-UMSC, IV-UMSC and IA-IV-UMSC, Fig. 10C shows the analysis results of IA-ADSC, IV-ADSC and IA-IV-ADSC, and Fig. 10D shows the analysis results of IA-BMSC, IV-BMSC and IA-IV-BMSC. Data are expressed as mean ± SEM, * represents p< 0.05 and ** represents p< 0.01.

The results in Fig. 10A and Fig. 10B show that significant reduction of fibrosis is observed in IA-IV-UMSC compared to IV-UMSC, IA-UMSC group and the control group. As shown in the results in Fig. 10C, significant reduction of fibrosis is observed in IA-IV-ADSC compared to IV-ADSC, IA-ADSC group and the control group. The results in Fig. 10D also show that significant reduction of fibrosis is observed in IA-IV-BMSC compared to IV-BMSC, IA-BMSC group and the control group. The results indicate that the transplantation strategy of the therapeutic cell of the present disclosure can enhance the ability of the therapeutic cell to reduce the fibrosis following the myocardial infarction.

To sum up, the present disclosure provides the novel transplantation strategy, in which the pharmaceutical composition including the therapeutic cell is administered to the subject with the ischemia condition of the tissue. The first dose of the pharmaceutical composition is administered by the intra-arterial injection, and then the second dose of the pharmaceutical composition is administered by the intra-venous injection, which can maintain the survival state and immunomodulatory ability of the therapeutic cell after the transplantation, so as to overcome the ischemia condition of the tissue and the hypoxic environment of spleen cells to activate the immune system, and can allow the therapeutic cell to have excellent clinical translation to improve the functional damage caused by the ischemia condition of the tissue. The results in the specification indicate that the transplantation strategy of the therapeutic cell of the present disclosure is not only safe and effective, but also enables the therapeutic cell to play an important role in rescuing ischemic brain injury and cardiac ischemic injury.

## Claims

1. A pharmaceutical composition for use in treatment of an ischemia condition of a tissue, wherein the pharmaceutical composition comprises a therapeutic cell, which therapeutic cell is a mesenchymal stem cell, a first dose of the pharmaceutical composition is administered by an intra-arterial injection, and then a second dose of the pharmaceutical composition is administered by an intra-venous injection, wherein an administration time interval between the first dose of the pharmaceutical composition and the second dose of the pharmaceutical composition is 2-4 hours.

2. The pharmaceutical composition for use of claim 1, wherein the mesenchymal stem cell is an umbilical cord mesenchymal stem cell, a bone marrow mesenchymal stem cell or an adipose mesenchymal stem cell.

3. The pharmaceutical composition for use of claims 1 or 2, wherein the first dose of the pharmaceutical composition is administered 24 hours after the ischemia condition of the tissue occurs.

4. The pharmaceutical composition for use of any one of claims 1 to 3, wherein the tissue is a brain.

5. The pharmaceutical composition for use of claim 4, wherein the ischemia condition is an ischemic stroke.

6. The pharmaceutical composition for use of claims 4 or 5, wherein the first dose of the pharmaceutical composition comprises at least 1×10⁵ of the therapeutic cells, and the second dose of the pharmaceutical composition comprises at least 1×10⁶ of the therapeutic cells.

7. The pharmaceutical composition for use of any one of claims 1 to 3, wherein the tissue is a heart.

8. The pharmaceutical composition for use of claim 7, wherein the ischemia condition is a myocardial infarction.

9. The pharmaceutical composition for use of claims 7 or 8, wherein the first dose of the pharmaceutical composition comprises at least 0.5×10⁶ of the therapeutic cells, and the second dose of the pharmaceutical composition comprises at least 1×10⁶ of the therapeutic cells.

## Patentansprüche

1. Pharmazeutische Zusammensetzung zur Verwendung bei der Behandlung eines Ischämieleidens eines Gewebes, wobei die pharmazeutische Zusammensetzung eine therapeutische Zelle, bei der es sich um eine mesenchymale Stammzelle handelt, umfasst, eine erste Dosis der pharmazeutischen Zusammensetzung durch eine intraarterielle Injektion verabreicht wird und dann eine zweite Dosis der pharmazeutischen Zusammensetzung durch eine intravenöse Injektion verabreicht wird, wobei ein Verabreichungszeitintervall zwischen der ersten Dosis der pharmazeutischen Zusammensetzung und der zweiten Dosis der pharmazeutischen Zusammensetzung 2-4 Stunden beträgt.

2. Pharmazeutische Zusammensetzung zur Verwendung nach Anspruch 1, wobei es sich bei der mesenchymalen Stammzelle um eine mesenchymale Nabelschnurstammzelle, eine mesenchymale Knochenmarkstammzelle oder eine mesenchymale Fettstammzelle handelt.

3. Pharmazeutische Zusammensetzung zur Verwendung nach Anspruch 1 oder 2, wobei die erste Dosis der pharmazeutischen Zusammensetzung 24 Stunden nach dem Auftreten des Ischämieleidens des Gewebes verabreicht wird.

4. Pharmazeutische Zusammensetzung zur Verwendung nach einem der Ansprüche 1 bis 3, wobei es sich bei dem Gewebe um ein Gehirn handelt.

5. Pharmazeutische Zusammensetzung zur Verwendung nach Anspruch 4, wobei es sich bei dem Ischämieleiden um einen ischämischen Schlaganfall handelt.

6. Pharmazeutische Zusammensetzung zur Verwendung nach Anspruch 4 oder 5, wobei die erste Dosis der pharmazeutischen Zusammensetzung mindestens 1×10⁵ therapeutische Zellen umfasst und die zweite Dosis der pharmazeutischen Zusammensetzung mindestens 1×10⁶ therapeutische Zellen umfasst.

7. Pharmazeutische Zusammensetzung zur Verwendung nach einem der Ansprüche 1 bis 3, wobei es sich bei dem Gewebe um ein Herz handelt.

8. Pharmazeutische Zusammensetzung zur Verwendung nach Anspruch 7, wobei es sich bei dem Ischämieleiden um einen Myokardinfarkt handelt.

9. Pharmazeutische Zusammensetzung zur Verwendung nach Anspruch 7 oder 8, wobei die erste Dosis der pharmazeutischen Zusammensetzung mindestens 0,5×10⁶ therapeutische Zellen umfasst und die zweite Dosis der pharmazeutischen Zusammensetzung mindestens 1×10⁶ therapeutische Zellen umfasst.

## Revendications

1. Composition pharmaceutique pour utilisation dans le traitement d'un état ischémique d'un tissu, dans laquelle la composition pharmaceutique comprend une cellule thérapeutique, laquelle cellule thérapeutique est une cellule souche mésenchymateuse, une première dose de la composition pharmaceutique est administrée par injection intra-artérielle, et ensuite une deuxième dose de la composition pharmaceutique est administrée par injection intra-veineuse, dans laquelle un intervalle de temps d'administration entre la première dose de la composition pharmaceutique et la deuxième dose de la composition pharmaceutique est de 2 à 4 heures.

2. Composition pharmaceutique pour utilisation selon la revendication 1, dans laquelle la cellule souche mésenchymateuse est une cellule souche mésenchymateuse du cordon ombilical, une cellule souche mésenchymateuse de moelle osseuse ou une cellule souche mésenchymateuse adipeuse.

3. Composition pharmaceutique pour utilisation selon la revendication 1 ou 2, dans laquelle la première dose de la composition pharmaceutique est administrée 24 heures après l'apparition de l'état ischémique du tissu.

4. Composition pharmaceutique pour utilisation selon l'une quelconque des revendications 1 à 3, dans laquelle le tissu est un cerveau.

5. Composition pharmaceutique pour utilisation selon la revendication 4, dans laquelle l'état ischémique est un accident vasculaire cérébral ischémique.

6. Composition pharmaceutique pour utilisation selon la revendication 4 ou 5, dans laquelle la première dose de la composition pharmaceutique comprend au moins 1×10⁵ des cellules thérapeutiques, et la deuxième dose de la composition pharmaceutique comprend au moins 1×10⁶ des cellules thérapeutiques.

7. Composition pharmaceutique pour utilisation selon l'une quelconque des revendications 1 à 3, dans laquelle le tissu est un cœur.

8. Composition pharmaceutique pour utilisation selon la revendication 7, dans laquelle l'état ischémique est un infarctus du myocarde.

9. Composition pharmaceutique pour utilisation selon la revendication 7 ou 8, dans laquelle la première dose de la composition pharmaceutique comprend au moins 0,5×10⁶ des cellules thérapeutiques, et la deuxième dose de la composition pharmaceutique comprend au moins 1×10⁶ des cellules thérapeutiques.
